# EUROPÄISCHE PATENTSCHRIFT

(11) **EP 3 110 481 B1**
(45) Veröffentlichungstag und Bekanntmachung des Hinweises auf die Patenterteilung: **31.10.2018**
(21) Anmeldenummer: 15702125.4
(22) Anmeldetag: 21.01.2015
(51) Int. Cl.: A61M 5/315, A61M 5/24

(54) **INJEKTIONSVORRICHTUNG ZUR VERABREICHUNG EINES FLUIDEN PRODUKTS**
INJECTION DEVICE FOR ADMINISTERING A LIQUID PRODUCT
DISPOSITIF D'INJECTION POUR ADMINISTRER UN PRODUIT FLUIDE

(30) Priorität: 26.02.2014 CH 275142014
(43) Veröffentlichungstag der Anmeldung: 04.01.2017
(73) Patentinhaber: Tecpharma Licensing AG, 3401 Burgdorf (CH)
(72) Erfinder: TSCHIRREN, Markus, CH-3400 Burgdorf (CH); HIRSCHEL, Jürg, CH-3007 Bern (CH)
(86) Internationale Anmeldenummer: PCT/CH2015/000005
(87) Internationale Veröffentlichungsnummer: WO 2015/127567

(56) Entgegenhaltungen:
- EP-A1- 2 351 591
- WO-A1-2013/178602
- DE-A1-102007 026 556

## Beschreibung

Die vorliegende Erfindung bezieht sich auf eine Injektionsvorrichtung zur Verabreichung eines fluiden Produkts, insbesondere eines Medikaments, wie beispielsweise Teriparatid zur Osteoporosetherapie.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus der WO2006/125329A1 ist eine Dosiervorrichtung eines Injektionsgeräts bekannt, mit welcher eine abzugebende Dosis variabel eingestellt werden kann. Die Dosiervorrichtung weist eine Drehhülse auf, die aus der Dosiervorrichtung zur Vorbereitung einer Dosisabgabe relativ zu einem Gehäuse der Injektionsvorrichtung herausgedreht werden kann.

Aus der WO2008/019517A1 ist ein Fix-Dose Injektionsgerät bekannt, wobei nur durch Drehung des Dosierknopfs eine abzugebende Dosis eingestellt werden kann.

Aus der EP2351591A1, der DE102007026556A1 und der WO2013/178602 sind ferner Injektionsvorrichtungen bekannt, wobei jeweils eine festgelegte Dosis eines fluiden Produkts aufdosiert und ausgeschüttet werden kann.

Es ist eine Aufgabe der vorliegenden Erfindung eine alternative Injektionsvorrichtung zur Aufdosierung und zur Ausschüttung einer festgelegten Dosis eines fluiden Produkts bereitzustellen, beziehungsweise mehrere festgelegte Dosen, das heisst eine erste, zweite und weitere bis zur letzten festgelegten Dosis, wobei die festgelegten Dosen gleich gross sind.

Diese Aufgabe wird durch den Gegenstand des unabhängigen Anspruchs gelöst. Vorteilhafte Ausführungsformen ergeben sich aus den abhängigen Ansprüchen.

Im Folgenden bedeutet distal eine Richtung zum injektionsnadelseitigen Ende der Injektionsvorrichtung und proximal zum dosierknopfseitigen Ende.

Die Erfindung betrifft eine Injektionsvorrichtung zur Aufdosierung und zur Ausschüttung einer festgelegten Dosis eines fluiden Produkts mit einer Gewindestange, wobei das Gewinde eine Zahnung aufweist. Ferner weist die Injektionsvorrichtung eine Rotationshülse und eine Führungshülse auf, wobei die Rotationshülse und die Führungshülse je ein Eingriffselement umfassen, welche in die Zahnung der Gewindestange eingreifen können. Es können auch mehrere Eingriffselemente an der Rotationshülse und an der Führungshülse angeordnet sein, die in die Zahnung der Gewindestange eingreifen können. Die Dosierhülse kann in einem Gewindeeingriff mit der Rotationshülse sein. Vorzugsweise ist der Gewindeeingriff als nicht selbsthemmende Gewindeverbindung ausgebildet. Ferner können die Rotationshülse und die Führungshülse relativ zueinander axial fest und drehbar angeordnet sein. Zudem kann die Rotationshülse relativ zu der Führungshülse und/oder zu der Dosierhülse und/oder Gewindestange drehbar angeordnet sein.

Um eine festgelegte Dosis des fluiden Produkts aufzudosieren, kann die Rotationshülse relativ zu der Führungshülse gedreht werden, bis die Drehbewegung durch einen ersten Anschlagkontakt zwischen der Rotationshülse und der Führungshülse begrenzt wird. Dazu kann die Rotationshülse in eine erste Drehrichtung gedreht werden. Die Dosierhülse kann zur Aufdosierung des fluiden Produkts aus der Injektionsvorrichtung axial herausziehbar sein, wohingegen die Dosierhülse zur Verabreichung des fluiden Produkts in die Injektionsvorrichtung axial hineinstossbar sein kann. Zur Verabreichung des fluiden Produkts kann daher die Rotationshülse in eine zweite Drehrichtung gedreht werden, bis die Drehbewegung durch einen zweiten Anschlagkontakt zwischen der Rotationshülse und der Führungshülse begrenzt wird. Der erste und der zweite Anschlagkontakt zwischen der Rotationshülse und der Führungshülse kann dadurch gebildet werden, dass die Rotationshülse einen radial nach aussen ragenden Vorsprung aufweist, der zwischen zwei an der Führungshülse vorgesehenen radial nach innen ragenden Vorsprüngen rotativ hin und her bewegbar ist. Alternativ umfasst die Rotationshülse zwei nach aussen ragende Vorsprünge, welche zwischen entsprechenden an der Führungshülse angebrachten radial nach innen ragenden Vorsprüngen rotativ hin und her bewegbar sind.

Das Eingriffselement der Rotationshülse und das Eingriffselement der Führungshülse können mit der Zahnung der Gewindestange derart zusammenwirken, dass eine Drehung der Gewindestange in eine Drehrichtung zugelassen und in die Gegendrehrichtung gesperrt werden kann. Zur Verabreichung des fluiden Produkts aus der Injektionsvorrichtung ist die Drehung der Gewindestange zulässig, wohingegen zur Aufdosierung des fluiden Produkts gesperrt wird. Die Gewindestange kann mittels Gewindeverbindung mit der Führungshülse oder alternativ mit einem Gehäuse der Injektionsvorrichtung verbunden sein. Die Führungshülse kann mit dem Gehäuse axial fest und drehfest verbunden sein oder alternativ einstückig mit dem Gehäuse ausgebildet sein.

Die Eingriffselemente der Rotationshülse und der Führungshülse können als elastische Federarme ausgebildet sein, wobei die Eingriffselemente in Umfangsrichtung an den entsprechenden Hülsen angebracht sein können. Die Eingriffselemente der Rotationshülse und der Führungshülse können einen Kopf mit einer steilen und einer flachen Flanke aufweisen. Diese Köpfe können mittels Federarmen elastisch in die Zahnung der Gewindestange eingreifen. Der Eingriffsmechanismus der Eingriffselemente der Rotationshülse und der Führungshülse in die Zahnung der Gewindestange kann als Ratschenmechanismus ausgebildet sein. Dazu kann die Gewindestange in Längsrichtung mehrere Längskerbungen oder Längsnuten aufweisen, die mindestesten einen Zahn, vorzugsweise mehrere Zähne mit einer oder mehreren Zahnlücken formen. Die Längskerbung oder Längsnut oder Zahn oder Zahnlücke weisen eine steile und eine flache Flanke auf.

Die festgelegte Dosis kann einerseits durch die Drehpositionen, welche zwischen dem ersten und zweiten Anschlagkontakt zwischen den Anschlägen der Rotationshülse und der Führungshülse definiert sind, und andererseits durch die Gewindesteigung zwischen der Rotationshülse und der Dosierhülse beziehungsweise dem axialen Weg der Dosierhülse festgelegt werden. Alternativ oder zusätzlich kann der axiale Weg der Dosierhülse durch axiale Anschläge und Gegenanschläge begrenzt werden. Dazu kann die Dosierhülse zwei axial beabstandete Anschläge aufweisen.

Die Injektionsvorrichtung kann eine Übersetzung oder Untersetzung der Aufziehbewegung der Dosierhülse beziehungsweise der Ausschüttbewegung der Gewindestange vorsehen. Dazu können die Gewindesteigung des Gewindeeingriffs zwischen der Führungshülse oder dem Gehäuse und der Gewindestange und die Gewindesteigung des Gewindeeingriffs zwischen der Dosierhülse und der Rotationshülse unterschiedlich sein. Vorzugsweise ist die Gewindesteigung des Gewindeeingriffs zwischen der Dosierhülse und der Rotationshülse grösser als die Gewindesteigung des Gewindeeingriffs zwischen der Führungshülse oder dem Gehäuse und der Gewindestange. Alternativ kann die Gewindesteigung des Gewindeeingriffs zwischen der Führungshülse oder dem Gehäuse und der Gewindestange grösser oder gleich sein als die Gewindesteigung des Gewindeeingriffs zwischen der Dosierhülse und der Rotationshülse.

Vorzugsweise weist die Rotationshülse und die Führungshülse je ein Eingriffselement auf und die Gewindestange vier Längskerbungen oder Längsnuten beziehungsweise vier Längszähne mit vier Zahnlücken auf, die in Umfangsrichtung um etwa 90 Grad versetzt zueinander angeordnet sind. Alternativ umfasst die Rotationshülse zwei Eingriffselemente, die um etwa 180 Grad in Umfangsrichtung versetzt zueinander an der Rotationshülse angeordnet sind, und die Führungshülse weist zwei Eingriffselemente auf, die ebenfalls um etwa 180 Grad in Umfangsrichtung versetzt zueinander an der Führungshülse angebracht sind. Alternativ können jeweils vier Eingriffselemente an der Rotationshülse oder Führungshülse vorgesehen sein, die um etwa 90 Grad in Umfangsrichtung versetzt zueinander angeordnet sind.

Alternativ können die gleiche Anzahl von Eingriffselemente an der Rotationshülse und an der Führungshülse angebracht sein, wie die Anzahl der Längskerbungen oder Längsnuten der Gewindestange beziehungsweise die Anzahl der Längszähne oder Zahnlücken der Gewindestange vorgesehen sind.

Vorzugsweise umfasst die Injektionsvorrichtung zwei in Umfangsrichtung versetzt angeordnete Anschlagkontakte zwischen der Rotationshülse und der Führungshülse, wobei der Abstand zwischen den beiden Anschlägen etwa dem Abstand zwischen zwei benachbarten Einrastpositionen des Eingriffelements der Rotationshülse und/oder der Führungshülse in die Längskerbung oder Längsnut oder Zahnlücke der Gewindestange entspricht.

Die Eingriffselemente der Rotationshülse und der Führungshülse und die Zahnung der Gewindestange können derart ausgebildet sein, dass beim Aufdosieren und bei der Ausschüttung des fluiden Produkts ein oder mehrere Klick-Geräusche erzeugt werden kann.

Aufgrund der Gewindeverbindung zwischen der Dosierhülse, welche drehfest und axial bewegbar in dem Gehäuse der Injektionsvorrichtung gelagert sein kann, und der Rotationshülse, welche axial fest und drehbar in dem Gehäuse der Injektionsvorrichtung vorgesehen sein kann, kann die Rotationshülse beim Aufdosieren und beim Ausschütten des fluiden Produkts je in eine Drehbewegung in Aufdosier- und Ausschüttrichtung gelangen.

Beim Aufdosieren des fluiden Produkts kann die Gewindestange mit Hilfe des Eingriffs zwischen dem Eingriffselement der Führungshülse und der Zahnung Gewindestange relativ zu dem Gehäuse der Injektionsvorrichtung axial fest und drehfest gehalten werden. Dazu gelangen eine steile Flanke des Kopfs des Eingriffelements der Führungshülse und eine steile Flanke der Längskerbung oder Längsnut oder Zahn oder Zahnlücke in Anschlagkontakt. Ferner kann beim Aufdosieren des fluiden Produkts das Eingriffselement der Rotationshülse ausser Eingriff mit der Zahnung der Gewindestange gelangen. Dabei gleitet eine flache Flanke des Kopfs des Eingriffselements der Rotationshülse über eine flache Flanke der Längskerbung oder der Längsnut oder Zahn oder Zahnlücke der Gewindestange und gelangt in die nächste Längskerbung oder Längsnut oder Zahnlücke der Gewindestange.

Beim Ausschütten des fluiden Produkts kann die Rotationshülse aufgrund eines Anschlags zwischen einer steilen Flanke des Kopfs des Eingriffselements der Rotationshülse und einer steilen Flanke der Längskerbung oder der Längsnut oder Zahn der Gewindestange die Drehbewegung auf die Gewindestange übertragen. Dabei gleitet eine flache Flanke des Kopfs des Eingriffselements der Führungshülse über eine flache Flanke der Längskerbung oder Längsnut oder Zahn oder Zahnlücke der Gewindestange. Die Gewindestange kann beim Ausschütten der festgelegten Dosis aufgrund der Gewindeverbindung zwischen Gewindestange und der Führungshülse oder alternativ dem Gehäuse sich in die distale Richtung schrauben. Dabei gleitet das Eingriffselement, insbesondere der Kopf oder ein Teil des Kopfs der Rotationshülse axial entlang der Längskerbung oder Längsnut der Gewindestange. Die Gewindestange bewegt sich axial relativ zu dem Gehäuse der Injektionsvorrichtung in die distale Richtung und stösst über einen Flansch einen Stopfen in die distale Richtung. Der Flansch kann axial fest und vorzugsweise drehbar am distalen Ende der Gewindestange befestigt sein. Der Stopfen in einer Karpule kann das fluide Produkt über eine an der Karpule oder an einem die Karpule aufnehmenden Karpulenhalter befestigten Injektionsnadel herauspressen.

Zum Schutz der Karpule oder des die Karpule aufnehmenden Karpulenhalters kann die Injektionsvorrichtung eine Verschlusskappe aufweisen. Die Verschlusskappe kann axial fest und vorzugsweise drehfest mit dem Gehäuse der Injektionsvorrichtung verbunden sein. Die Verschlusskappe kann von der Injektionsvorrichtung abgenommen werden, um vor dem Gebrauch der Injektionsvorrichtung eine Injektionsnadel an die Karpule anzubringen, indem eine Kanüle der Injektionsnadel ein Septum der Karpule durchstossen kann, um eine Fluidverbindung zwischen der Injektionsnadel und der Karpule zu bilden.

Die Injektionsvorrichtung kann einen Dosierknopf vorsehen, welcher axial fest und vorzugsweise drehfest am proximalen Ende der Dosierhülse angebracht sein kann. Der Dosierknopf kann einstückig mit der Dosierhülse gebildet sein.

Die Injektionsvorrichtung kann zudem einen Mechanismus vorsehen, der ein Einstellen einer weiteren Dosis verhindern kann, wenn die letzte Dosis ausgeschüttet worden ist.

Dazu kann die Rotationshülse der Injektionsvorrichtung einen Anschlag aufweisen, welcher gegen einen an der Gewindestange vorgesehenen Anschlag anstösst. Somit kann durch einen axialen und/oder radialen Anschlagkontakt zwischen der Rotationshülse und der Gewindestange das Einstellen oder Aufziehen einer weiteren Dosis verhindert werden, wenn die letzte Dosis ausgeschüttet worden ist. Vorzugweise können zwei Anschläge an der Rotationshülse vorgesehen sein, die in Anschlagkontakt mit entsprechenden zwei an der Gewindestange angebrachten Anschläge kommen können und so eine Drehbewegung der Rotationshülse in Aufdosierrichtung verhindern.

In einem Ausführungsbeispiel kann die Dosierhülse zwischen der Rotationshülse und der Führungshülse angeordnet sein. In einem anderen Ausführungsbeispiel können die Führungshülse und die Dosierhülse koaxial in der Injektionsvorrichtung angeordnet sein. Die Gewindeverbindung zwischen der Rotationshülse und der Dosierhülse kann als eine eingängige oder mehrgängige Gewindeverbindung oder als Segmente einer Gewindeverbindung ausgebildet sein.

Ferner kann die Injektionsvorrichtung eine Anzeigevorrichtung umfassen, welche die Anfangsposition, die aufgezogene Position und/oder die ausgeschüttete Position der Dosierhülse oder des Einstellknopfs anzeigen kann. Die Anzeigevorrichtung kann als eine visuelle, akustische und/oder taktile Anzeige ausgebildet sein. Die visuelle Anzeigevorrichtung kann eine Markierung und/oder ein Symbol und/oder eine Anzeigeziffer umfassen.

Dazu kann die Injektionsvorrichtung eine Anzeigehülse mit einer Anzeigevorrichtung aufweisen. Vorzugsweise ist die Anzeigehülse mit der Dosierhülse einstückig verbunden. Alternativ kann die Dosierhülse eine Anzeigevorrichtung aufweisen. Ferner kann die Dosierhülse eine visuelle Anzeigevorrichtung in Form einer Bedruckung und/oder eines Aufklebers aufweisen.

Die Erfindung wird im Folgenden an mehreren Figuren beschrieben. Die hierbei offenbarten Merkmale bilden je einzeln und in Kombination die Erfindung vorteilhaft weiter. Es zeigen:
Figur 1: eine Explosionsansicht einer ersten Ausführungsform einer Injektionsvorrichtung
Figur 2a: eine Längsschnittansicht der ersten Ausführungsform der Injektionsvorrichtung in einer Anfangsposition einer Dosierhülse (4)
Figur 2b: die in Figur 2a gezeigte Injektionsvorrichtung in Queransicht, wobei die Queransicht der in Figur 2a eingezeichneten Querschnittlinie A-A entspricht
Figur 2c: die in Figur 2a gezeigte Injektionsvorrichtung in Queransicht, wobei die Queransicht der in Figur 2a eingezeichneten Querschnittlinie B-B entspricht
Figur 3a: eine Längsschnittansicht der ersten Ausführungsform der Injektionsvorrichtung in einer aufgezogenen Position der Dosierhülse (4)
Figur 3b: die in Figur 3a gezeigte Injektionsvorrichtung in Queransicht, wobei die Queransicht der in Figur 2a eingezeichneten Querschnittlinie C-C entspricht
Figur 3c: die in Figur 3a gezeigte Injektionsvorrichtung in Queransicht, wobei die Queransicht der in Figur 2a eingezeichneten Querschnittlinie D-D entspricht
Figur 4a: eine Längsschnittansicht der ersten Ausführungsform der Injektionsvorrichtung nach Ausschüttung der letzten Dosis
Figur 4b: die in Figur 4a gezeigte Injektionsvorrichtung in Detailansicht, wobei die Detailansicht der in

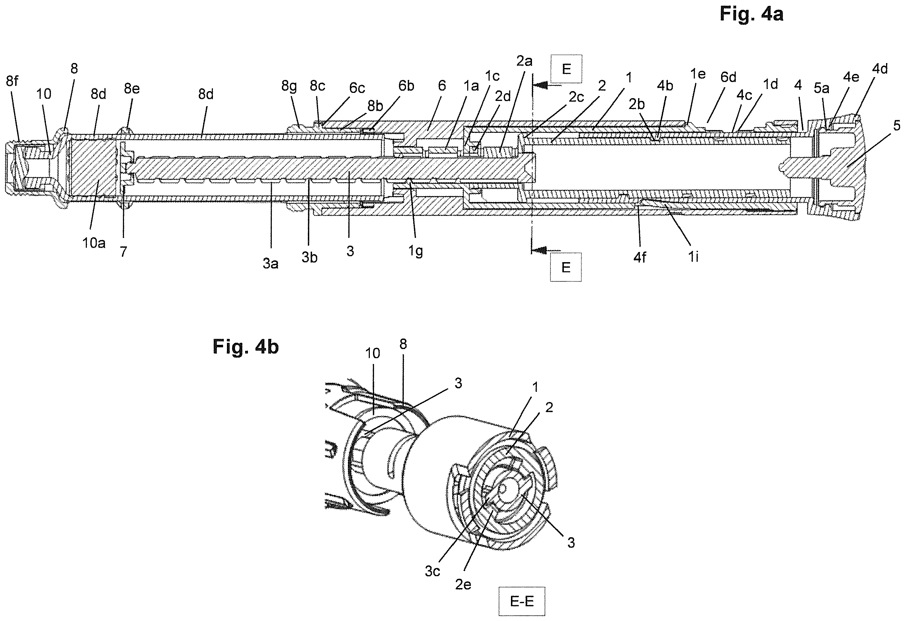

Figur 4a eingezeichneten Querschnittlinie E-E entspricht, wobei ein Teil des Gehäuses (6) ausgespart ist, sodass das Innere der Injektionsvorrichtung ersichtlich ist

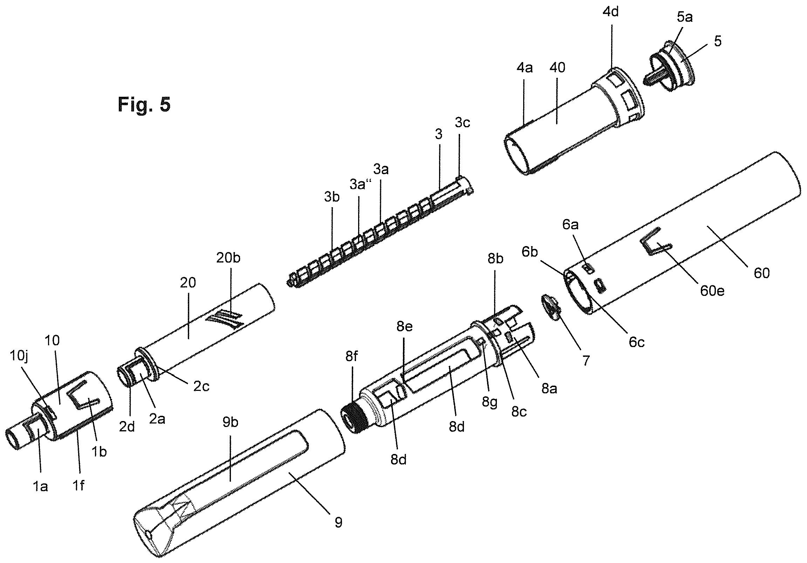

Figur 5: eine Explosionsansicht einer zweiten Ausführungsform einer Injektionsvorrichtung

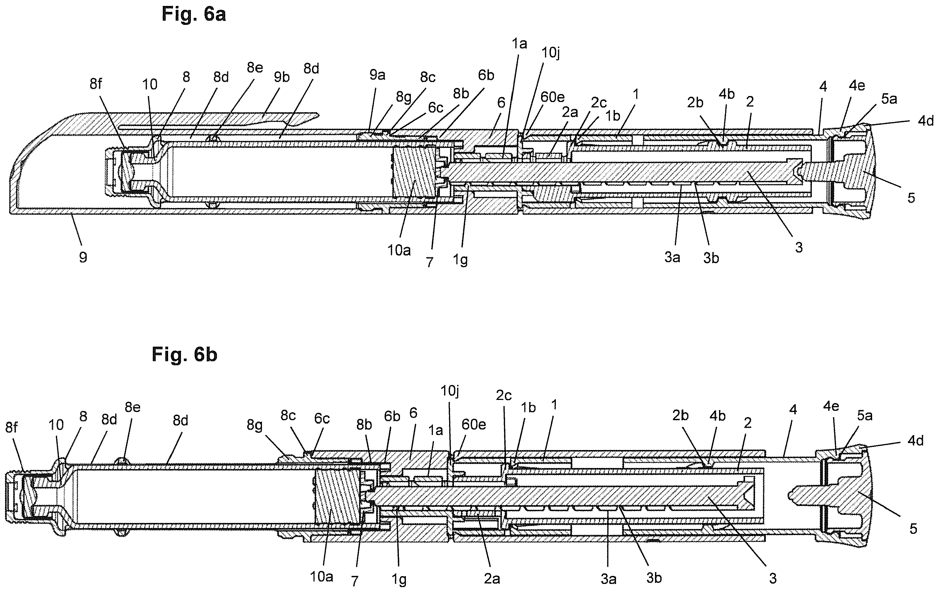

Figur 6a. eine Längsschnittansicht der zweiten Ausführungsform der Injektionsvorrichtung in einer Anfangsposition einer Dosierhülse (4)
Figur 6b: eine Längsschnittansicht der zweiten Ausführungsform der Injektionsvorrichtung in einer aufgezogenen Position der Dosierhülse (4)

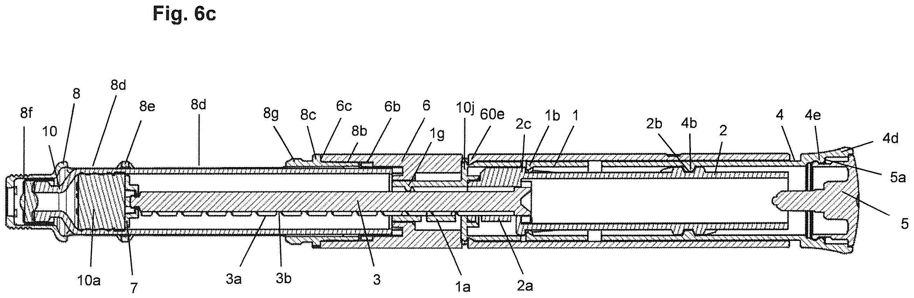

Figur 6c: eine Längsschnittansicht der zweiten Ausführungsform der Injektionsvorrichtung nach Ausschüttung der letzten Dosis

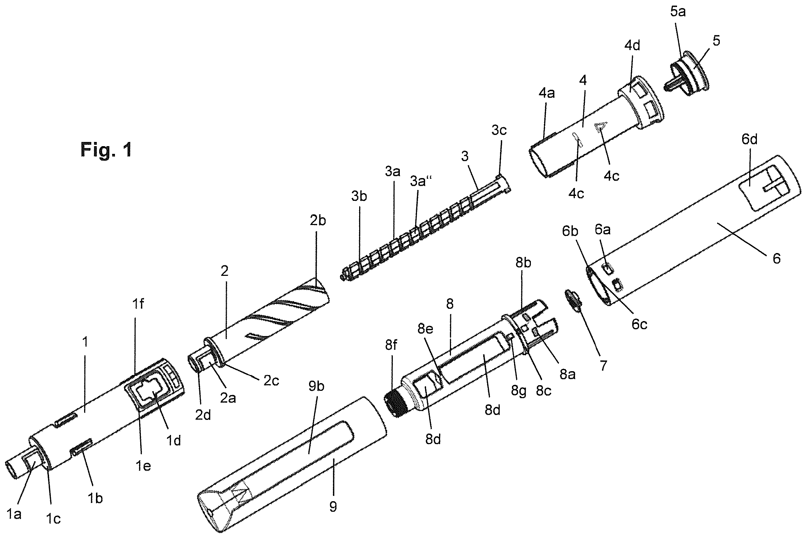

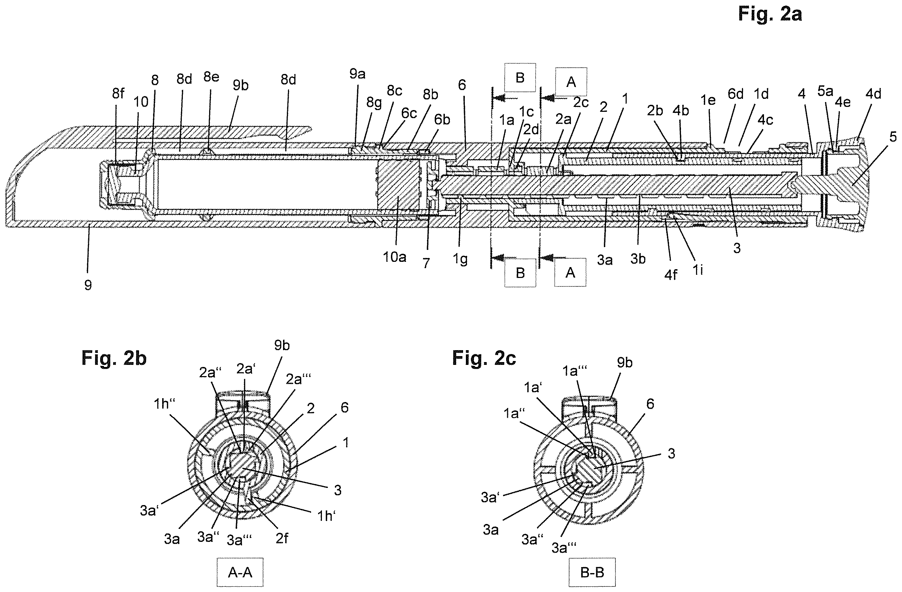

In der Figur 1 ist eine Explosionsansicht einer ersten Ausführungsform einer erfindungsgemässen Injektionsvorrichtung dargestellt. Die Injektionsvorrichtung umfasst einen Karpulenhalter (8), welcher eine Karpule (beispielsweise in Figur 2a; 10) aufnehmen kann, wobei der Karpulenhalter (8) über eine Karpulenhalternase (8a) mit einer distalen Gehäusenasenaussparung (6a) eines Gehäuses (6) axial fest verbunden ist. Ferner ist an dem Karpulenhalter (8) ein Karpulenhaltersteg (8b) zur drehfesten Verbindung über eine korrespondierende Gehäusenut (6b) mit dem Gehäuse (6) vorgesehen. Ein Karpulenhalteranschlag (8c) kann axial an eine distale Gehäusekante (6c) des Gehäuses (6) anschlagen. Eine Karpulenhalteraussparung (8d) in dem Karpulenhalter (8) dient zur Anzeige der relativen Position eines in der Karpule (beispielsweise in Figur 2a; 10) axial bewegbaren Stopfens (beispielsweise in Figur 2a; 10a). Die Karpulenhalteraussparung (8d) ist durch einen Karpulenhalterquersteg (8e) unterbrochen, wobei die Karpulenhalteraussparung (8d) in zwei Teile geteilt ist. Falls sich der Stopfen (beispielsweise in Figur 4a; 10a) in dem distaleren angeordneten Teil der Karpulenhalteraussparung (8d) befindet, entnimmt der Benutzer beispielsweise die Information, dass die letzte Dosis aus der Injektionsvorrichtung ausgeschüttet worden ist und keine neue Dosis aufgezogen werden kann. An dem distalen Ende des Karpulenhalters (8) ist eine Nadelverbindungselement (8f) vorgesehen, welches zur lösbaren Befestigung einer Injektionsnadel (nicht ersichtlich) vorgesehen ist. Die Injektionsvorrichtung umfasst ferner eine Verschlusskappe (9), welche über eine lösbare Verbindung mit dem Karpulenhalter (8) verbindbar ist. Dazu weist der Karpulenhalter (8) einen Karpulenhaltervorsprung (8g) auf, der eine Ringnut (beispielsweise in Figur. 2a; 9a) der Verschlusskappe (9) einrasten kann. Die Verschlusskappe (9) kann vorzugsweise einen Verschlusskappenclip (9b) umfassen. Vorzugsweise weist die Ringnut (beispielsweise in Figur 2a; 9a) der Verschlusskappe (9) eine Vertiefung auf, sodass die Verschlusskappe (9) relativ zu dem Karpulenhalter (8) drehfest anordbar ist. Ferner umfasst die Injektionsvorrichtung eine Führungshülse (1), welche über einen Formschluss mit einem Gehäuse (6) der Injektionsvorrichtung axial fest und drehfest verbunden ist. Dazu weist das Gehäuse (6) ein Gehäusefenster (6d) auf, welches ein um ein Führungshülsenfenster (1d) radial nach aussen ragenden Führungshülsenfensterrahmen (1e) aufnimmt. Ferner ist an der Führungshülse (1) ein Führungshülsensteg (1f) angeordnet, der mit einer Nut (nicht ersichtlich) in dem Gehäuse (6) eine drehfeste Verbindung bildet. Die Führungshülse (1) weist ein Eingriffselement (1a) auf, welches mit der Zahnung der Gewindestange (3) zusammenwirken kann. Die Injektionsvorrichtung umfasst ferner eine Rotationshülse (2), die axial fest und drehbar mit der Führungshülse (1) verbunden ist. Dazu weist die Rotationshülse (2) einen Rotationshülsenringsteg (2c) auf, der axial an den Führungshülsenhaltearm (1b), vorzugsweise an zwei Führungshülsenhaltearme (1b) anschlagen kann, wobei ferner die Rotationshülsenkante (2d) einen axialen Anschlagkontakt mit einer Führungshülsenstirnseite (1c) der Führungshülse (1) bilden kann. Die Rotationshülse (2) umfasst ebenfalls ein Eingriffselement (2a), welches mit der Zahnung der Gewindestange (3) zusammen wirken kann. Die Zahnung der Gewindestange (3) umfasst einen Zahn (3a), vorzugsweise mehrere Zähne, besonders bevorzugt vier Zähne. Ferner weist die Gewindestange (3) ein Aussengewinde (3b) auf, welches mit einem Innengewinde (beispielsweise in Figur 2a; 1g) der Führungshülse eine Gewindeverbindung bildet. Der Eingriffsmechanismus der Eingriffselemente (2a, 1a) der Rotationshülse (2) und der Führungshülse (1) in die Zahnung der Gewindestange (3) ist als Ratschenmechanismus ausgebildet. Die Eingriffselemente (2a, 1a) der Rotationshülse (2) und der Führungshülse (1) wirken mit der Zahnung der Gewindestange (3) derart zusammen, dass beim Aufdosieren der festgelegten Dosis und beim Ausschütten der festgelegten Dosis ein Klick-Geräusch erzeugt wird. Um ein Einstellen einer weiteren Dosis zu verhindern, wenn die letzte Dosis ausgeschüttet worden ist, weist die Rotationshülse (2) einen Rotationshülsenanschlag (beispielsweise in Figur 4b; 2e) auf, der in Anschlagkontakt mit einem an der Gewindestange (3) vorgesehenen Gewindestangenanschlag (3c) kommen kann. Die Injektionsvorrichtung umfasst ferner eine Dosierhülse (4). Die Dosierhülse (4) ist über einen Dosierhülsensteg (4a) mit einer Führungshülsennut (nicht ersichtlich) der Führungshülse (1) relativ zu dem Gehäuse (6) der Injektionsvorrichtung drehfest und axial bewegbar gelagert. Der Benutzer kann die Dosierhülse (1) mit Hilfe eines am proximalen Ende der Dosierhülse (1) angeordneten Dosierhülsengriffs (4d) axial hin und her bewegen. Zur Anzeige der einzelnen Dosierbewegungen weist die Dosierhülse (1) vorzugweise eine Anzeige (4c) auf. Am proximalen Ende der Dosierhülse (1) kann zudem ein Dosierknopf (5) angebracht sein, der über eine Dosierknopfnut (5a) mit einem Dosierhülsenringsteg (4e) der Dosierhülse (1) axial fest verbunden ist. Die Dosierhülse (1) ist über eine Gewindeverbindung mit der Rotationshülse (2) verbunden. Die Innenseite der Dosierhülse (4) umfasst ein Dosierhülsengewinde (beispielsweise in Figur 2a; 4b), welches in Eingriff mit dem Rotationshülsengewinde (2b) an der Aussenseite der Rotationshülse (2) ist.

In der Figur 2a ist eine Längsschnittansicht der ersten Ausführungsform der Injektionsvorrichtung in einer Anfangsposition einer Dosierhülse (4) dargestellt. Die im Gehäuse (6) axial bewegbar und drehfest gelagerte Dosierhülse (4) ist in dem Injektionsgerät eingeschoben, wobei die an der Dosierhülse (4) angebrachte Anzeige (4c) durch das Führungshülsenfenster (1d) und durch das Gehäusefenster (6d) ersichtlich ist und dem Benutzer anzeigt, dass die Injektionsvorrichtung in der Anfangsposition ist. Die Dosierhülse (4) ist über das Dosierhülsengewinde (4b) mit dem Rotationshülsengewinde (2b) der Rotationshülse (2) in Gewindeeingriff. Wie in Figur 2b ersichtlich, befindet sich ein radial nach aussen ragender Vorsprung (2f) der Rotationshülse (2) in Anschlagkontakt mit einem ersten radial nach innen ragenden Vorsprung (1h') der Führungshülse (1). Ein an dem Eingriffselement (2a) der Rotationshülse (2) angebrachter Kopf (2a') ist im Eingriff mit der Zahnung der Gewindestange (3). Dazu greift der Kopf (2a') des Eingriffselements (2a) der Rotationshülse (2) zwischen zwei benachbarte Zähne (3a) in eine Zahnlücke (3a'") der Gewindestange (3), nämlich zwischen einer steilen Flanke (3a') eines Zahns (3a) und einer flachen Flanke (3a") eines benachbarten Zahns (3a) der Gewindestange (3). Ferner ist, wie in Figur 2c dargestellt, ein an dem Eingriffselement (1a) der Führungshülse (1) vorgesehener Kopf (1a') ebenfalls im Eingriff mit der Zahnung der Gewindestange (3). Dazu greift der Kopf (1a') des Eingriffselements (1a) der Führungshülse (1) ebenfalls zwischen zwei benachbarte Zähne (3a) in eine Zahnlücke (3a'") der Gewindestange (3). Der Kopf (1a') des Eingriffselements (1a) der Führungshülse befindet sich zwischen einer steilen Flanke (3a') eines Zahns (3a) und einer flachen Flanke (3a") eines benachbarten Zahns (3a) der Gewindestange (3). Damit die relative Position zwischen der Dosierhülse (4) und dem Führungselement (1) definiert ist, ist ein radial nach innen vorgespannter Führungshülsenschnapper (1i) der Führungshülse (1) in Eingriff mit einer an der Dosierhülse (4) vorgesehener Dosierhülsenaussparung (4f). Dieser Eingriff bildet einen Kraftschluss zwischen dem Führungshülsenschnapper (1i) und der Dosierhülsenaussparung (4f). Ferner kann dieser Eingriff ein Spiel zwischen der Dosierhülse (4), der Rotationshülse (2), der Gewindestange (3) und der Führungshülse (1) aufheben.

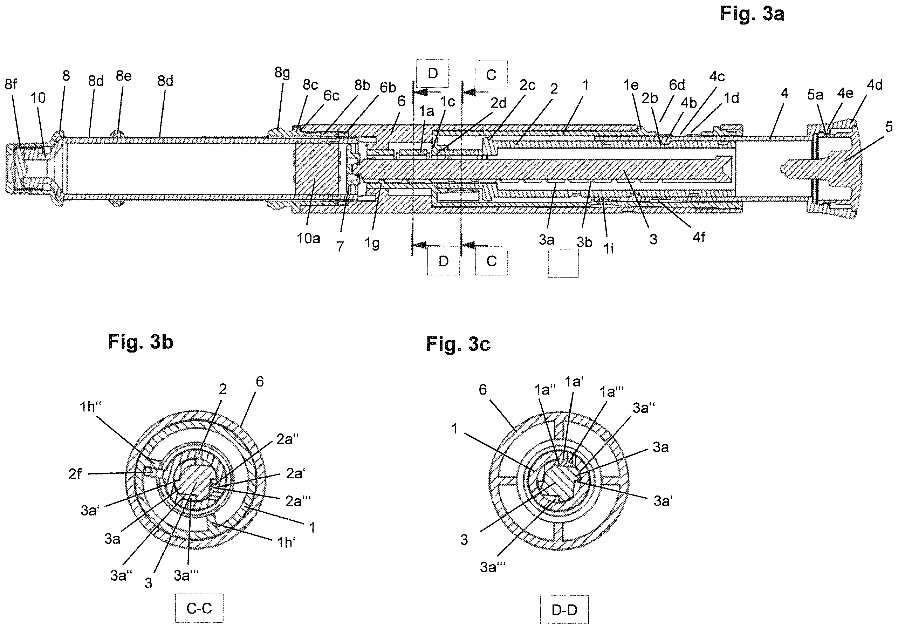

In der Figur 3a ist eine Längsschnittansicht der ersten Ausführungsform der Injektionsvorrichtung in einer aufgezogenen Position der Dosierhülse (4) dargestellt. Um eine Dosis einzustellen oder aufzuziehen, zieht der Benutzer den Dosierhülsengriff (4d) in die proximale Richtung. Die Dosierhülse (4) wird dabei axial in die proximale Richtung verschoben. Der Führungshülsenschnapper (1i) der Führungshülse (1) gleitet ausser Eingriff mit der Dosierhülsenaussparung (4f) der Dosierhülse (4). Aufgrund des Gewindeeingriffs zwischen der Dosierhülse (4) und der Rotationshülse (2), dreht die Rotationshülse (2) in eine erste Drehrichtung bis der Vorsprung (2f) der Rotationshülse (2) in Anschlagkontakt mit einem zweiten radial nach innen ragenden Vorsprung (1h") der Führungshülse (1) in Anschlagkontakt gelangt, wie in Figur 3b dargestellt ist. Die flache Flanke (2a'") des Kopfs (2a') des Eingriffselements (2a) der Rotationshülse (2) gleitet dabei über die flache Flanke (3a") des Zahns (3a) der Gewindestange (3) und gelangt dabei in eine benachbarte Zahnlücke (3a'"). Wie in Figur 3c ersichtlich ist, sind die steile Flanke (1a") des Kopfs (1a') des Eingriffselements (1a) der Führungshülse (1) in Anschlagkontakt mit der steilen Flanke (3a') des Zahns (3a) der Gewindestange (3), sodass die Gewindestange (3) rotativ fest gehalten wird. Die an der Dosierhülse (4) angebrachte Anzeige (4c) zeigt dem Benutzer durch das Führungshülsenfenster (1d) und durch das Gehäusefenster (6d) an, dass die Injektionsvorrichtung in der aufgezogenen Position ist.

Die Aufziehbewegung der Dosierhülse (4) und die Ausschüttbewegung der Gewindestange (3) ist übersetzt. Die Gewindesteigung der Gewindeverbindung (4b, 2b) zwischen der Dosierhülse (4) und der Rotationshülse (2) ist grösser als die Gewindesteigung der Gewindeverbindung (3b, 1g) zwischen der Gewindestange (3) und der Führungshülse (1).

Um eine festgelegte Dosis auszuschütten, drückt der Benutzer den Dosierknopf (5) in die distale Richtung, wobei die Dosierhülse (4) in die distale Richtung axial verschoben wird. Die Rotationshülse (2) dreht in die der ersten Drehrichtung entgegengesetzte Drehrichtung bis der Vorsprung (2f) der Rotationshülse (2) in Anschlagkontakt mit dem ersten radial nach innen ragenden Vorsprung (1h') der Führungshülse (1) in Anschlagkontakt gelangt, wie in Figur 2b dargestellt ist. Die steile Flanke (2a") des Kopfs (2a') des Eingriffselements (2a) der Rotationshülse (2) ist in Anschlagkontakt mit der steilen Flanke (3a') des Zahns (3a) der Gewindestange (3) und überträgt ein Drehmoment auf die Gewindestange (3). Die Gewindestange (3) schraubt sich dadurch aufgrund der Gewindeverbindung zwischen dem Innengewinde (1g) der Führungshülse (1) und dem Aussengewinde (3b) der Gewindestange (3) in die distale Richtung. Dabei gleitet der Kopf (2a') des Eingriffslements (2a) der Rotationshülse (2) axial entlang der Zahnlücke (3a"') der Gewindestange (3). Die flache Flanke des Kopfs (1a''') der Führungshülse (1) gleitet über die flache Flanke des Zahns (3a") der Gewindestange. Dabei wird über einen an der Gewindestange (3) axial fest angebrachten Flansch (7) ein von einer Karpule (10) aufgenommenen Stopfen (10a) in die distale Richtung gestossen. Der Stopfen (10a) kann das fluide Produkt über eine an dem Karpulenhalter befestigten Injektionsnadel (nicht ersichtlich) herauspressen.

In der Figur 4a ist eine Längsschnittansicht der ersten Ausführungsform der Injektionsvorrichtung nach Ausschüttung der letzten Dosis dargestellt. Nachdem die letzte Dosis aus der Injektionsvorrichtung ausgeschüttet worden ist, befindet sich der Stopfen (10a) in dem distal angeordneten Teil der Karpulenhalteraussparung (8d), welcher durch den Karpulenhalterquersteg (8e) in zwei Teile geteilt ist. Der Benutzer entnimmt somit die Information, dass die letzte Dosis aus der Injektionsvorrichtung ausgeschüttet worden ist und keine neue Dosis ausgezogen werden kann. Wie in Figur 4b gezeigt, sind zwei Rotationshülsenanschläge (2e) der Rotationshülse (2) in Anschlagkontakt mit den entsprechenden zwei Gewindestangenanschläge (3c) der Gewindestange (3). Es kann somit keine weitere Dosis über die Dosierhülse (4) aufgezogen werden, da eine Rotation der Rotationshülse (2) blockiert wird.

In der Figur 5 ist eine Explosionsansicht einer zweiten Ausführungsform einer erfindungsgemässen Injektionsvorrichtung dargestellt. Die Injektionsvorrichtung unterscheidet sich von der Injektionsvorrichtung der ersten Ausführungsform im Wesentlichen nur in Bezug auf die Ausgestaltung der Führungshülse (10), der Dosierhülse (40), der Rotationshülse (20) und des Gehäuses (60). Die Führungshülse (10) ist mit Gehäuse (60) über einen an der Führungshülse (10) vorgesehenen Führungshülsensteg (1f) und an dem Gehäuse (60) angebrachte Nut (6b) drehfest und über einen nach innen ragenden Führungshülsenvorsprung (10j) der Führungshülse (10) und einen an dem Gehäuse (60) angebrachten Gehäusehaltearm (60e) axial fest verbunden. Die Rotationshülse (20) umfasst ein Aussengewinde, welches in einer Gewindeverbindung mit einem Innengewinde der Dosierhülse (40) ist. Das Aussengewinde der Rotationshülse (20) ist als Rotationshülsengewinde oder als Rotationshülsengewindesegment (20b) und das Innengewinde der Dosierhülse (40) als Dosierhülsengewinde oder als Dosierhülsengewindesegment (40b) ausgebildet. Ferner weisen die Führungshülse (10) und das Gehäuse (60) kein Fenster auf, durch welches eine Anzeige ersichtlich ist. Die Dosierhülse (40) kann keine Anzeige oder alternativ eine Anzeige in Form einer Markierung, beispielsweise in Form eines Strichs (nicht ersichtlich) aufweisen, wobei die Markierung in einer Anfangsposition der Dosierhülse (40) durch das Gehäuse (60) verdeckt wird und in einer aufgezogenen Position der Dosierhülse (40) ersichtlich ist. In Bezug auf das Zusammenwirken der restlichen Teile der zweiten Ausführungsform der erfindungsgemässen Injektionsvorrichtung wird auf die Injektionsvorrichtung der ersten Ausführungsform gemäss Figuren 1 und deren Beschreibung verwiesen.

In der Figur 6a ist eine Längsschnittansicht der zweiten Ausführungsform der Injektionsvorrichtung in einer Anfangsposition einer Dosierhülse (4) gezeigt und in der Figur 6b ist eine Längsschnittansicht der zweiten Ausführungsform der Injektionsvorrichtung in einer aufgezogenen Position der Dosierhülse (4) dargestellt. Ferner zeigt Figur 6c eine Längsschnittansicht der zweiten Ausführungsform der Injektionsvorrichtung nach Ausschüttung der letzten Dosis. Die Figuren 6a, 6b und 6c zeigen das Zusammenwirken der Führungshülse (10), der Rotationshülse (20), der Gewindestange (3) der Doserhülse (40), des Dosierknopfs (5), des Gehäuses (60), des Flanschs (7), des Karpulenhalters (8), der Verschlusskappe (9), der Karpule (9) und des Stopfens (10a) gemäss der in Figuren 2a, 2b, 2c, 3a, 3b, 3c, 4a und 4b gezeigten ersten Ausführungsform. Somit wird auf die Funktionsbeschreibung der ersten Ausführungsform gemäss der in Figuren 2a, 2b, 2c, 3a, 3b, 3c, 4a und 4b und deren Beschreibungen verwiesen, welche auf die zweite Ausführungsform der Injektionsvorrichtung mit Ausnahme der in Figur 5 erwähnten Unterschiede zu übertragen ist.

### Bezugszeichen:

- 1; 10: Führungshülse
1a Eingriffselement
1a' Kopf
1a" steile Flanke des Kopfs
1a'" flache Flanke des Kopfs
1b Führungshülsenhaltearm
1c Führungshülsenstirnseite
1d Führungshülsenfenster
1e Führungshülsenfensterrahmen
1f Führungshülsensteg
1g Innengewinde
1h' erster Vorsprung
1h" zweiter Vorsprung
1i Führungshülsenschnapper
10j Führungshülsenvorsprung
- 2; 20: Rotationshülse
2a Eingriffselement
2a' Kopf
2a" steile Flanke des Kopfs
2a"' flache Flanke des Kopfs
2b Rotationshülsengewinde
20b Rotationshülsengewinde / -segment
2c Rotationshülsenringsteg
2d Rotationshülsenkante
2e Rotationshülsenanschlag
2f Vorsprung
- 3: Gewindestange
3a Zahn
3a' steile Flanke des Zahns
3a" flache Flanke des Zahns
3a'" Zahnlücke
3b Aussengewinde
3c Gewindestangeanschlag
- 4; 40: Dosierhülse
4a Dosierhülsensteg
4b Dosierhülsengewinde
40b Dosierhülsengewinde / -segment
4c Anzeige
4d Dosierhülsengriff
4e Dosierhülsenringsteg
4f Dosierhülsenaussparung
- 5: Dosierknopf
5a Dosierknopfringnut
- 6; 60: Gehäuse
6a Gehäusenasenaussparung
6b Gehäusenut
6c Gehäusekante
6d Gehäusefenster
60e Gehäusehaltearm
- 7: Flansch
- 8: Karpulenhalter
8a Karpulennase
8b Karpulenhaltersteg
8c Karpulenhalteranschlag
8d Karpulenhalteraussparung
8e Karpulenhalterquersteg
8f Nadelverbindungselement
8g Karpulenhaltervorsprung
- 9: Verschlusskappe
9a Ringnut
9b Verschlusskappenclip

- 10: Karpule
10a Stopfen

## Patentansprüche

1. Injektionsvorrichtung zur Aufdosierung und zur Ausschüttung einer festgelegten Dosis eines fluiden Produkts mit
- einer Gewindestange (3), wobei das Gewinde eine Zahnung aufweist,
- einer Rotationshülse (2; 20) mit einem Eingriffselement (2a), wobei das Eingriffselement (2a) in die Zahnung der Gewindestange (3) eingreifbar ist,
- eine Führungshülse (1) mit einem Eingriffselement (1a), wobei das Eingriffselement (1a) in die Zahnung der Gewindestange (3) eingreifbar ist,
- einer Dosierhülse (4; 40), welche relativ zu der Rotationshülse (2; 20) und relativ zu der Führungshülse (1; 10) axial bewegbar angeordnet ist, **dadurch gekennzeichnet, dass**
die Dosierhülse (4; 40) in einem Gewindeeingriff (4b, 2b) mit der Rotationshülse (2; 20) ist.

2. Injektionsvorrichtung nach Anspruch 1, **dadurch gekennzeichnet, dass** die Gewindestange (3) in einem Gewindeeingriff (3b, 1g) mit der Führungshülse oder einem Gehäuse der Injektionsvorrichtung ist.

3. Injektionsvorrichtung nach Anspruch 2, **dadurch gekennzeichnet, dass** die Gewindesteigung des Gewindeeingriffs der Führungshülse oder dem Gehäuse und der Gewindestange und der Gewindesteigung des Gewindeeingriffs der Dosierhülse und der Rotationshülse unterschiedlich sind.

4. Injektionsvorrichtung nach Anspruch 3, **dadurch gekennzeichnet, dass** die Gewindesteigung des Gewindeeingriffs zwischen der Dosierhülse und der Rotationshülse grösser ist als die Gewindesteigung des Gewindeeingriffs zwischen der Führungshülse oder dem Gehäuse und der Gewindestange.

5. Injektionsvorrichtung nach Anspruch 3, **dadurch gekennzeichnet, dass** die Gewindesteigung des Gewindeeingriffs zwischen der Führungshülse oder dem Gehäuse und der Gewindestange grösser oder gleich ist als die Gewindesteigung des Gewindeeingriffs zwischen der Dosierhülse und der Rotationshülse.

6. Injektionsvorrichtung nach einem der vorhergehenden Ansprüche, **dadurch gekennzeichnet, dass** die Rotationshülse (2; 20) und die Führungshülse (1; 10) relativ zueinander axial fest angeordnet sind.

7. Injektionsvorrichtung nach einem der vorhergehenden Ansprüche, **dadurch gekennzeichnet, dass** die Rotationshülse (2) und die Führungshülse (1; 10) relativ zueinander drehbar angeordnet sind.

8. Injektionsvorrichtung nach Anspruch 7, **dadurch gekennzeichnet, dass** die Drehbewegung zwischen der Rotationshülse (2; 20) und der Führungshülse (1; 10) durch einen Anschlagkontakt zwischen der Rotationshülse (2; 20) und der Führungshülse (1; 10) begrenzt wird.

9. Injektionsvorrichtung nach einem der vorhergehenden Ansprüchen, **dadurch gekennzeichnet, dass** die Rotationshülse (2; 20) und die Dosierhülse (4; 40) relativ zueinander drehbar angeordnet sind.

10. Injektionsvorrichtung nach einem der vorhergehenden Ansprüche, **dadurch gekennzeichnet, dass** die Rotationshülse (2; 20) und die Gewindestange (3) relativ zueinander drehbar angeordnet sind.

11. Injektionsvorrichtung nach Anspruch 10, **dadurch gekennzeichnet, dass** die Drehbewegung zwischen der Rotationshülse (2; 20) und der Gewindestange (3) durch einen Anschlagkontakt zwischen der Rotationshülse (2; 20) und der Gewindestange (3) begrenzt wird.

12. Injektionsvorrichtung nach einem der vorhergehenden Ansprüche, **dadurch gekennzeichnet, dass** die Dosierhülse (4; 40) zur Aufdosierung des fluiden Produkts aus der Injektionsvorrichtung axial herausziehbar ist und dass die Dosierhülse (4; 40) zur Verabreichung des fluiden Produkts in die Injektionsvorrichtung axial hereinstossbar ist.

13. Injektionsvorrichtung nach einem der vorhergehenden Ansprüche, **dadurch gekennzeichnet, dass** der axiale Weg der Dosierhülse (4; 40) durch axiale Anschläge und Gegenanschläge begrenzt wird.

14. Injektionsvorrichtung nach einem der vorhergehenden Ansprüche, **dadurch gekennzeichnet, dass** das Eingriffselement der Rotationshülse (2; 20) und das Eingriffselement der Führungshülse (1; 10) mit der Zahnung der Gewindestange (3) derart zusammenwirken, dass eine Drehung der Gewindestange (3) in eine Drehrichtung zugelassen wird und in die Gegendrehrichtung gesperrt wird.

15. Injektionsvorrichtung nach einem der vorhergehenden Ansprüche, **dadurch gekennzeichnet, dass** die Dosierhülse (4; 40) zwischen der Rotationshülse (2; 20) und der Führungshülse (1; 10) angeordnet ist.

## Claims

1. An injection device for dose setting and for dispensing a fixed dose of a fluid product comprising
- a threaded rod (3), the thread having a tooth arrangement,
- a rotary sleeve (2; 20) having an engagement element (2a), wherein the engagement element (2a) is engageable into the tooth arrangement of the threaded rod (3),
- a guide sleeve (1) having an engagement element (1a), wherein the engagement element (1a) is engageable into the tooth arrangement of the threaded rod (3), and
- a dosing sleeve (4; 40) which is arranged axially moveably relative to the rotary sleeve (2; 20) and relative to the guide sleeve (1; 10), **characterised in that**
the dosing sleeve (4; 40) is in threaded engagement (4b, 2b) with the rotary sleeve (2; 20).

2. An injection device according to claim 1 **characterised in that** the threaded rod (3) is in threaded engagement (3b, 1g) with the guide sleeve or a housing of the injection device.

3. An injection device according to claim 2 **characterised in that** the thread pitch of the threaded engagement of the guide sleeve or the housing and the threaded rod and the thread pitch of the threaded engagement of the dosing sleeve and the rotary sleeve are different.

4. An injection device according to claim 3 **characterised in that** the thread pitch of the threaded engagement between the dosing sleeve and the rotary sleeve is greater than the thread pitch of the threaded engagement between the guide sleeve or the housing and the threaded rod.

5. An injection device according to claim 3 **characterised in that** the thread pitch of the threaded engagement between the guide sleeve or the housing and the threaded rod is greater than or equal to the thread pitch of the threaded engagement between the dosing sleeve and the rotary sleeve.

6. An injection device according to one of the preceding claims **characterised in that** the rotary sleeve (2; 20) and the guide sleeve (1; 10) are arranged axially fixed relative to each other.

7. An injection device according to one of the preceding claims **characterised in that** the rotary sleeve (2) and the guide sleeve (1; 10) are arranged rotatably relative to each other.

8. An injection device according to claim 7 **characterised in that** the rotary movement between the rotary sleeve (2; 20) and the guide sleeve (1; 10) is limited by an abutment contact between the rotary sleeve (2; 20) and the guide sleeve (1; 10).

9. An injection device according to one of the preceding claims **characterised in that** the rotary sleeve (2; 20) and the dosing sleeve (4, 40) are arranged rotatably relative to each other.

10. An injection device according to one of the preceding claims **characterised in that** the rotary sleeve (2; 20) and the threaded rod (3) are arranged rotatably relative to each other.

11. An injection device according to claim 10 **characterised in that** the rotary movement between the rotary sleeve (2; 20) and the threaded rod (3) is limited by an abutment contact between the rotary sleeve (2; 20) and the threaded rod (3).

12. An injection device according to one of the preceding claims **characterised in that** the dosing sleeve (4; 40) can be pulled axially out of the injection device for dose setting of the fluid product and that the dosing sleeve (4; 40) can be pushed axially into the injection device for administration of the fluid product.

13. An injection device according to one of the preceding claims **characterised in that** the axial travel of the dosing sleeve (4; 40) is limited by axial abutments and counterpart abutments.

14. An injection device according to one of the preceding claims **characterised in that** the engagement element of the rotary sleeve (2; 20) and the engagement element of the guide sleeve (1; 10) cooperate with the tooth arrangement of the threaded rod (3) in such a way that rotation of the threaded rod (3) is permitted in one direction of rotation and is blocked in the opposite direction of rotation.

15. An injection device according to one of the preceding claims **characterised in that** the dosing sleeve (4; 40) is arranged between the rotary sleeve (2; 20) and the guide sleeve (1; 10).

## Revendications

1. Dispositif d'injection pour le dosage et la distribution d'une dose définie d'un produit fluide avec
- une tige filetée (3), dans lequel le filetage présente une denture,
- une douille de rotation (2 ; 20) avec un élément de mise en prise (2a), dans lequel l'élément de mise en prise (2a) peut être mis en prise dans la denture de la tige filetée (3),
- une douille de guidage (1) avec un élément de mise en prise (1a), dans lequel l'élément de mise en prise (1a) peut être mis en prise dans la denture de la tige filetée (3),
- une douille de dosage (4 ; 40), qui est agencée de manière axialement mobile par rapport à la douille de rotation (2 ; 20) et par rapport à la douille de guidage (1 ; 10), **caractérisé en ce que**
la douille de dosage (4 ; 40) est dans une prise par filetage (4b, 2b) avec la douille de rotation (2 ; 20).

2. Dispositif d'injection selon la revendication 1, **caractérisé en ce que** la tige filetée (3) est dans une prise par filetage (3b, 1g) avec la douille de guidage ou un boîtier du dispositif d'injection.

3. Dispositif d'injection selon la revendication 2, **caractérisé en ce que** le pas de filetage de la prise par filetage de la douille de guidage ou du boîtier et de la tige filetée et le pas de filetage de la prise par filetage de la douille de dosage et de la douille de rotation sont différents.

4. Dispositif d'injection selon la revendication 3, **caractérisé en ce que** le pas de filetage de la prise par filetage entre la douille de guidage et la douille de rotation est supérieur au pas de filetage de la prise par filetage entre la douille de guidage ou le boîtier et la tige filetée.

5. Dispositif d'injection selon la revendication 3, **caractérisé en ce que** le pas de filetage de la prise par filetage entre la douille de guidage ou le boîtier et la tige filetée est supérieur ou égal au pas de filetage de la prise par filetage entre la douille de dosage et la douille de rotation.

6. Dispositif d'injection selon l'une quelconque des revendications précédentes, **caractérisé en ce que** la douille de rotation (2 ; 20) et la douille de guidage (1 ; 10) sont agencées de manière axialement fixe l'un par rapport à l'autre.

7. Dispositif d'injection selon l'une quelconque des revendications précédentes, **caractérisé en ce que** la douille de rotation (2) et la douille de guidage (1 ; 10) sont agencées de manière rotative l'une par rapport à l'autre.

8. Dispositif d'injection selon la revendication 7, **caractérisé en ce que** le mouvement de rotation entre la douille de rotation (2 ; 20) et la douille de guidage (1 ; 10) est limité par un contact de butée entre la douille de rotation (2 ; 20) et la douille de guidage (1 ; 10).

9. Dispositif d'injection selon l'une quelconque des revendications précédentes, **caractérisé en ce que** la douille de rotation (2 ; 20) et la douille de guidage (4 ; 40) sont agencées de manière rotative l'une par rapport à l'autre.

10. Dispositif d'injection selon l'une quelconque des revendications précédentes, **caractérisé en ce que** la douille de rotation (2 ; 20) et la tige filetée (3) sont agencées de manière rotative l'une par rapport à l'autre.

11. Dispositif d'injection selon la revendication 10, **caractérisé en ce que** le mouvement de rotation entre la douille de rotation (2 ; 20) et la tige filetée (3) est limité par un contact de butée entre la douille de rotation (2 ; 20) et la tige filetée (3).

12. Dispositif d'injection selon l'une quelconque des revendications précédentes, **caractérisé en ce que** la douille de dosage (4 ; 40) peut être retirée axialement du dispositif d'injection pour le dosage du produit fluide et que la douille de dosage (4 ; 40) peut être poussée axialement dans le dispositif d'injection pour l'administration du produit fluide.

13. Dispositif d'injection selon l'une quelconque des revendications précédentes, **caractérisé en ce que** le trajet axial de la douille de dosage (4 ; 40) est limité par des butées axiales et contre-butées.

14. Dispositif d'injection selon l'une quelconque des revendications précédentes, **caractérisé en ce que** l'élément de mise en prise de la douille de rotation (2 ; 20) et l'élément de mise en prise de la douille de guidage (1 ; 10) coopèrent avec la denture de la tige filetée (3) de telle sorte qu'une rotation de la tige filetée (3) est autorisée dans un sens de rotation et bloquée dans le sens de rotation opposé.

15. Dispositif d'injection selon l'une quelconque des revendications précédentes, **caractérisé en ce que** la douille de dosage (4 ; 40) est agencée entre la douille de rotation (2 ; 20) et la douille de guidage (1 ; 10).
